# EUROPEAN PATENT SPECIFICATION

(11) **EP 0 923 937 B1**
(45) Date of publication and mention of the grant of the patent: **10.03.2004**
(21) Application number: 98890364.7
(22) Date of filing: 07.12.1998
(51) Int. Cl.: A61K 35/78, A61P 17/02

(54) **A HERBAL FORMULATION USEFUL AS A THERAPEUTIC AND COSMETIC APPLICATION FOR THE TREATMENT OF GENERAL SKIN DISORDERS**
KRÄUTER FORMULIERUNG MIT THERAPEUTISCHER UND KOSMETISCHER VERWENDUNG ZUR BEHANDLUNG VON ALLGEMEINEN HAUTERKRANKUNGEN
UNE FORMULATION A BASE DE PLANTES UTILISABLE DANS UNE APPLICATION THERAPEUTIQUE ET COSMETIQUE POUR LE TRAITEMENT D'AFFECTIONS DERMATOLOGIQUES EN GENERAL

(30) Priority: 08.12.1997 IN 351997
(43) Date of publication of application: 23.06.1999
(73) Proprietor: Council of Scientific and Industrial Research, New Delhi 110 001 (IN)
(72) Inventor: Prakash, Vaman Rao Diwan, Hyderabad-500007 (IN); Bhamidipalli, Subrahmanya Sitaramam, Hyderabad-500007 (IN); Sistla, Ramakrishna, Hyderabad-500007 (IN); Kondapuram, Vijaya Raghavan, Hyderabad-500007 (IN)
(74) Representative: Schwarz, Albin, Dr.

(56) References cited:
- PRAKASH V. DIWAN ET ALO.: "INFLUENCE OF TRIDAX PROCUMBENS ON WOUND HEALING" THE INDIAN JOURNAL OF MEDICAL RESEARCH, vol. 75, March 1982, pages 460-464, XP002101811
- PATENT ABSTRACTS OF JAPAN vol. 014, no. 057 (C-0684), 2 February 1990 & JP 01 284333 A (T HASEGAWA CO LTD), 15 November 1989
- DATABASE WPI Section Ch, Week 9717 Derwent Publications Ltd., London, GB; Class B04, AN 97-180065 XP002101812 & CN 1 092 995 A (MI C), 5 October 1994
- PAUL HEINZ LIST: 'ARZNEIFORMENLEHRE', 1985, WISSENSCHAFTLICHE VERLAGSGESELLSCHAFT MBH, STUTTGART
- LEUNG FOSTER: 'Encyclopedia of common natural ingredients used in food, drug and cosmetics', 1996, JOHN WILEY & SONS, INC.

## Description

The present invention relates to a herbal cream formulation useful for therapeutic and cosmetic applications: cracked heels, dry skin disorder, skin allergies, depigmentation and anti fungal activity. In the formulation of the present invention, the herbs that are used are known to possess anti-inflammatory, antiallergic and wound healing properties. The formulation may also be useful in treating chopped hands, hyperkeratosis and minor cuts and burn wounds.

### Background of the invention

Skin acts as a barrier between body and its environment maintaining a controlled dynamic equilibrium. Stresses imposed by environment can cause changes in skin and major purpose of creams under consideration is to help reverse these changes and maintain a normal healthy skin. Cracking of heels is a common problem, which is observed in almost all individuals in extreme winter and summer seasons in tropical climates. Skin is a protein gel hydrated in its inner most layers and largely dehydrated in its surface layer. The purpose of water in the outer corneous layer of skin is of great physiological importance in that (1) certain amount of desiccation from skin surface minimises bacterial multiplication and assists in maintaining integrity. (2) The continual desquamation of skin is nature's method of removing debris and foreign matter, which are generally embedded in skin apertures.

It is not desirable that drying of skin layer should proceed to such an extent that corneous layer prematurely flakes away and cracks develop on the skin surface.

Cracks on the heels, in extreme winter season, develop because of the closure of the pores in the outermost layers. This problem worsens if the atmosphere has low humidity. Cracks on heels are also developed in extreme summer seasons because of migration of water from innermost layers and subsequent evaporation from outer skin surface in low humid environment. In both cases, it is necessary to maintain the integrity of the skin by balancing water content in skin layers and to promote healing of fissures that develop upon prolonged exposure.

### Prior art discussion

In the market, at present, a number of formulations are available for cracked heels. The composition in all the preparations are different and some of them contain synthetic drugs like Salicylic acid as one of the ingredients. All the preparations are required to be used continuously for better performance of healing of the cracks developed on heels. There are some preparations, which are used for skin rashes and fungal infections between fingers and toes. Following are the preparations available with their ingredients. "Vaseline Heal Guard" of Ponds India ltd. contains the active ingredients: Salicylic Acid IP-1.5w/w, Lactic Acid-8.0w/w, Triclosan-0.1w/w and Cream base-QS. "Krack" of Paras Pharmaceuticals, Kalol contains: Dodishak and Sarjaras-ab35.4%, Raktapuraka-ab3.6%, Base to make 100%. "Lichensa" by Dollar Co.P. Ltd. Chennai, contains: Clotrimazole-0.50%w/w, Menthol-1.0%w/w, Ichthammol IP66-0.20%w/w, Boric acid-1.0%w/w, Zinc oxide-5.0%w/w and Lanolin base to -100%w/w., which is also claimed as stainfree. "Link ointment "by Link, Hyderabad contains: Madhuchist-19.5%, Erandi Thail-58.5%, Karpoor-2.5% and Base-19.5%.

### Summary of the invention

Accordingly, studies were undertaken to develop cream formulations containing herbal drugs and synthetic ingredients for topical application over the affected areas which will help healing of cracks on heels and also minor burn wounds and cuts on the skin. These formulations have proven usefulness in removal of pigmentation on surface of the skin and reducing the allergic reactions and can also be used in cosmetic therapy.

### Objects of the invention

The objective of the present invention is to provide a herbal formulation useful for therapeutic and cosmetic applications such as cracked heels, dry skin disorder, skin allergies, depigmentation and anti fungal activity, which obviates the drawbacks of the present formulations available in the market.

Another object of the present invention is to prepare a herbal cream using the formulations of the present invention.

### Detailed description of the invention

In the embodiment of the present invention, plant extracts used may be Gymnema sylvestrae, Ocimium sanctum, Gum olibanum, Tridax procumbens, Allium sativum, Aloe vera, Turmeric, from any part of the plant such as leaf, root, bark, natural exudation of the bark, flower, fruit, stem or branch.

In another embodiment of the present invention the known drugs having anti-inflammatory and wound healing property used may be such as Salicylic acid, Piroxicam, Diclofenac sodium.

In still another embodiment of the present invention the base used may be such as aqueous cream base (containing emulsifying ointment BP 1993, preservative and water) or gel base (containing propylene glycol, carbopol 934 and monoethanol amine).

In yet another embodiment of the present invention the preservatives used may be such as Methyl paraben, Propyl paraben, p- Chlorocresol.

In still another embodiment of the present invention the humectants used may be as Glycerine and Propylene glycol.

As a result of intensive study conducted by the inventors with the aim of achieving afore mentioned objectives, new processes for the preparation of cream formulations for topical use were developed employing herbal drugs which are from natural origin, incorporating them into cream bases along with synthetic materials which are known to possess water retaining properties.

Accordingly, the present investigation deals with cream and gel based topical formulations. Each formulation has been described in detail giving the formula of the ingredients along with method of preparation.

The first step in the preparation of these formulations involves a process for making the plant material suitable for incorporating into cream/gel bases. The specified portion of the plant is collected and dried under shade at room temperature in an enclosed room for 72 hours or more until the material gets dried. A specified amount of material is then extracted with solvents like n-hexane, chloroform, ethanol, methanol and water, in cold/hot condition. The choice of the solvent depends upon the type of material expected at the end of extraction process. Extraction process was carried out in a closed container immersing specified amount of the plant material in respective solvents for 72 hours. At the end of this stage, solvent is decanted and filtered if necessary to make it free from the plant debris. The solvent is then concentrated by evaporating under vacuum at room temperature. if the solvent used is water, then concentrated solution is freeze dried to obtain the final product in powder form. If the solvent used is a non-polar solvent then final product would be an oily and viscous substance with specific physico-chemical properties. This final product is made into a formulation intended for topical use by using it as an ingredient for making creams and gels. Suitable preservatives like methyl paraben, propyl paraben and p-chlorocresol have been used. Humectants such as propylene glycol and glycerol have been used in appropriate quantities.

One embodiment of the invention provides a herbal formulation useful as a therapeutic and cosmetic applications for the treatment of general skin disorders, said composition comprising the following ingredients:
(a) at least two plant extracts in the form of oil or powder or mixtures thereof, the said plants extracts being selected from the group consisting of *Gymnena sylvestrae* water extract 3 to 6wt.%; *Tridax procumbens* water extract 3 to 6wt.%; its methanolic extract 4 to 6wt.%, *Allium sativum* oilhexane extract 1 to 3wt.%; dried juice of Aloe vera 2 to 6wt.%; *Gum Olibanum* powder in the natural form 4 to 7wt.%; *Gum Olibanum* resinoid or its organic extract 3 to 8wt.%; and resinoid free *Gum Olibanum* meal 5 to 10 wt.%.;
(b) optionally, including any drug having anti-inflammatory and wound healing property or mixture thereof, the said drug being selected from the group consisting of Diclofenac sodium 1-3wt.%, Salicyclic acid 1 to 4 wt. %, Piroxicam 1 to 2 wt. % , Turmeric powder 0.1 to 1 wt. %;
(c) a gel base containing carbopol ranging between 1 to 4 wt. %, or an aqueous cream base containing emulsifying ointment ranging between 20 to 40 wt. %, preservatives ranging between 0.05 -0.3 % and a humecant ranging between 1-4 wt. %; and
(d) remaining water to make 100 wt. %.

The preservatives used in the above composition may be selected from the group consisting of methyl paraben, propyl paraben and p-chlorocresol. Methyl paraben and propyl paraben used as preservatives range between 0.1 -0.3wt. %, p-chlorocresol used may range between 0.05 -0.2%, gum olibanum resinoid hexane extract ranges between 4- 6 wt. %, gum olibanum resinoid methanol extract ranges between 3-8 wt. %, gum olibanum resinoid ethanol extract ranges between 4 -6 wt. %, glycerine used as humectant ranges between 1 -4 wt. % and propylene glycol used as humectant ranges between 1-3 wt. %..

The plant extracts used are obtained from plant material selected from *Gymnema sylvestrae* leaf (R. Br), *Gum olibanum, Tridax procumbens, Allium sativum, Aloe Vera*.

Another embodiment of the present invention provides a herbal formulation useful for enhancing healing of burns and wounds comprising the following ingredients.
(a) plant extracts in the form of powder or oil of medicinal plants, being *Tridax procumbens* water extract 3 to 6wt. % and *gum olibanum* powder in the natural form 4-7 wt. %;
(b) a gel base containing carbopol ranging between 1 to 4 wt. %, or an aqueous cream base containing emulsifying ointment ranging between 20 to 40wt. %, preservatives ranging between 0.05-0.3% and a humecant ranging between 1-4 wt. %;
( c) humectants selected from propylene glycol ranging between 1 to 3 wt. % and Glycerine ranging between 1 to 4wt. %; and
(d) the balance being water to make 100wt. %.

Yet another embodiment of the invention provides an antifungal herbal formulation comprising the following ingredients:
(a) plant extracts in the form of powder or oil of medicinal plants, being *Tridax procumbens* water extract 3 to 6 wt. % or *Gum Oilibanum* powder in the natural form 4 to 7 wt. %;
(b) optionally , any drug having anti-inflammatory and wound healing property or mixture thereof, the said drug being selected from drugs such as Diclofenac sodium 1-3 wt. %, Salicyclic acid 1 to 4 wt. %, Piroxicam 1 to 2wt. %, Turmeric powder 0.1 to 1 wt. %;
(c ) a gel base containing carbopol ranging between 1 to 4 wt. %, or an aqueous cream base containing emulsifying ointment ranging between 20 to 40 wt. %, preservatives ranging between 0.05-0.3% and humecant ranging between 1-4 wt. %.
(d) humectants selected from propylene glycol ranging between 1 to 3wt. %, and Glycerine ranging between 1 to 4 wt. %; and
(e) the balance being water to make 100 wt. %.

Still another embodiment of the invention provides an anti allergic herbal formulation comprising the following ingredients:
(a) plant extracts in the form of powder or oil of medicinal plants being *Tridax procumbens* water extract 3 to 6 wt. % and gum Olibanum powder in the natural form 4 to 7 wt. %;
(b) a gel base containing carbopol ranging between 1 to 4wt.%, or an aqueous cream base containing emulsifying ointment ranging between 20 to 40 wt. %, preservatives ranging between 0.05 -0.3% and a humecant ranging between 1-4 wt. %;
(c) humectants selected from propylene glycol ranging between 1 to 3wt. %, and Glycerine ranging between 1 to 4 wt. %; and
(d) the balance being water to make 100 wt. %.

One more embodiment of the invention provides a herbal formulation useful for cosmetic applications as moisturiser comprising the following ingredients:
(a) at least two plant extracts in the form of powder or oil of medicinal plants, being selected from Gymnena sylvestrae water extract 3 to 6 wt. % *Aloe vera* (dried juice) 2-6 wt.% *Tridax procumbens* water extract 3 to 6 wt. %, *Gum Olibanum* meal and *Gum Olibanum* powder;
(b) optionally any drug having anti-inflammatory and wound healing property or mixture thereof, the said drug being selected from group consisting of Diclofenac sodium 1-3 wt%, salicylic ocid 1 to 4 wt%, Piroxicam 1 to 2 wt%, Turmeric powder 0.1 to 1 wt. %;
( c) a gel base containing carbopol ranging between 1 to 4 wt.%, or an aqueous cream base containing emulsifying ointment ranging between 20 to 40 wt. %, preservatives ranging between 0.05 -0.3% and a humecant ranging between 1-4 wt. %;
(d) humectants selected from propylene glycol ranging between 1 to 3 wt. %, and Glycerine ranging between 1 to 4 wt. %; and
(e) the balance being water to make 100 wt. %.

Another preferred embodiment of the invention relates to a herbal formulation useful for therapeutic and cosmetic applications particularly discoloration comprising the following ingredients:
(a) plant extracts in the form of powder or oil of medicinal plants, being *Gum Olibanum* resinoid, *Tridax procumbens* water extract 3 to 6 wt. %, *Gum Olibanum* powder;
(b) a gel base containing carbopol ranging between 1 to 4 wt.%, or an aqueous cream base containing emulsifying ointment ranging between 20 to 40 wt. %, preservatives ranging between 0.05 -0.3% and a humecant ranging between 1-4 wt. %;
(c) humectants selected from propylene glycol ranging between 1 to 3wt. %, and Glycerine ranging between 1 to 4 wt. %; and
(d) the balance being water to make 100 wt. %.

### Brief description of the accompanying drawings

The following portion provides brief description of the accompanying drawings of the specification, as follows:
Figure 1 shows cracked heel of a patient before treatment.
Figure 2 shows heel of the patient of figure 1 after treatment with the herbal composition of the invention.
Figure 3 shows cracked sole of a patent before treatment.
Figure 4 shows sole of the patent of figure 3 after treatment with the herbal composition of the present invention.
Figure 5 shows cracked heel of another patient.
Figure 6 shows sole of the patient of figure after treatment.
Figure 7 shows cracked sole of a female patient before treatment.
Figure shows sole of the female patient of figure 7 after treatment.
Figure 9 shows a cracked toe of a patient before treatment.
Figure 10 shows the toe of figure 9 after treatment with the present herbal composition.
Figure 11 shows sole of a patent having fungal infection before treatment.
Figure 12 shows the sole of the patent of figure 11 after treatment with the herbal composition of the present invention.

The following examples are given by way of illustration and these should not be construed to limit the scope of the present invention.

The following are to be noted while making these formulations.
1. All the ingredients are expressed in % w/w basis.
2. Emulsifying ointment used in these formulations is prepared as per the procedure given in the official compendium (British Pharmacopoeia, 1993).

### EXAMPLE - 1

| | |
|---|---|
| Tridax procumbens leaf extract | 5% |
| Carbopol 934 | 3% |
| Methyl paraben | 0.15% |
| Propyl paraben | 0.15% |
| Monoethanolamine | q.s to bring pH to 6 |
| Propylene glycol:water(50:50) | q.s to make 100% |

The leaves of Tridax procumbens were shade dried for 48 hours at room temperature. The crushed leaves (500g) were then soaked with water (one litre) for 72hours at room temperature. At the end of this period, water is decanted and then concentrated to 100ml by evaporating under vacuum at room temperature. This concentrated solution is then lyophilised to obtain powder.

Tridax procumbens leaf extract is dispersed in pure propylene glycol along with propyl paraben (0.15%). The mixture is thoroughly agitated to get a clear solution. Carbopol 934 is dispersed in propylene glycol and water (50:50) mixture along with methyl paraben in another vessel. The mixture is stirred continuously at 300 rpm for 2-3hours. Tridax procumbens solution was then added and stirring was continued for about 1 hour until a gel preparation is obtained. The pH of this gel is adjusted to 6 using monoethanolamine. The gel preparation obtained is clear, transparent and non-sticky.

### EXAMPLE - 2

| | |
|---|---|
| Gum Olibanum powder | 5% |
| Emulsifying ointment | 26% |
| Methyl paraben | 0.15% |
| Propyl paraben | 0.15% |
| Water | q.s to make 100% |

The naturally occurring Gum Olibanum exudate in dry state is taken as it is. The lumps (1 Kg) were powdered in an edge runner mill for 30 minutes. The powdered raw Gum olibanum was passed through 100 mesh sieve. Weighed quantity of the powder was dispersed in appropriate quantity of water along with methyl paraben (0.15%). Weighed quantity of emulsifying ointment is melted in another vessel and propyl paraben (0.15%) in dispersed in it (Oily phase). The dispersion containing Gum olibanum powder methyl paraben was also heated to the same temperature as that of emulsifying ointment. The aqueous dispersion containing Gum olibanum powder is added to the molten emulsifying ointment and the mixture is stirred continuously at 10,000 rpm for 1hour using homogeniser, to obtain cream consistency.

### EXAMPLE -3

| | |
|---|---|
| Gum olibanum powder | 6% |
| Tridax procumbens leaf extract | 4% |
| Emulsifying ointment | 25% |
| Propylene glycol | 2% |
| p-Chlorocresol | 0.1% |
| Water | q.s to make 100% |

The naturally occurring Gum Olibanum exudate in dry state is taken as it is. The lumps(1Kg) were powdered in an edge runner mill for 30 minutes. The powdered raw gum olibanum was passed through 100 mesh sieve.

The leaves of Tridax procumbens were shade dried for 48 hours at ambient temperature. The leaves (500g) were then soaked with water (1litre) for 72 hours at room temperature. At the end of this period, water is decanted and then concentrated to 100ml by evaporating under vacuum at room temperature. This concentrated solution is then lyophilised to obtain powder.

Weighed quantity of emulsifying ointment is taken in a tarred vessel along with p-chlorocresol and heated until the ointment melts. In a separate container, Gum Olibanum powder and Tridax procumbens leaf extract were dispersed in water. Required quantity of propylene glycol is also added. This aqueous mixture is heated to the same temperature as that of emulsifying ointment. The aqueous phase is then added to the oily phase, in hot condition and the mixture is stirred at 10,000 rpm for 1-3 hours until a cream consistency is obtained.

### EXAMPLE - 4

| | |
|---|---|
| Gymnema Sylvestrae Powder | 5% |
| Salicylic Acid | 2% |
| Gum Olibanum resinoid (n-Hexane extract) | 5% |
| Emulsifying ointment | 23% |
| Glycerol | 2% |
| p-Chlorolcresol | 0.1% |
| Water | q.s. to make 100% |

The leaves of Gymnema Sylvestrae were shade dried for 48hours at room temperature. The leaves were cut into small portions and immersed in water in a suitable vessel for 72 hours. At the end of this period, water is decanted, filtered. This aqueous portion is then concentrated by evaporating under vacuum at 45°C. this concentrated portion is lyophilised to obtain a powder.

Gum Olibanum lumps were powdered in 5 Kg lots in an edge runner mill for 30 minutes. The powdered raw Gum olibanum (250g) was then extracted at 30°C using n-hexane (1.5litres) as solvent in a vertical churner for 8 hours. The solvent containing resinoid was then decanted and was distilled off at atmospheric pressure. The resinoid (100g) was obtained which is pale yellow and having a refractive index of 1.5160, density 0.9297g/cm³ and surface tension 0.0290N/m at 30°C.

Weighed quantities of Gum Olibanum resinoid, p-chlorocresol and emusifying ointment are taken in a container and the mixture is heated under stirring until both resinoid and emulsifying ointment melt(oil phase). Gymnema sylvestrae powder, salicylic acid, glycerol are dispersed in water, in a suitable container and the mixture is kept for homogenisation until a homogenous dispersion is obtained. This dispersion is heated to the same temperature as that of oil phase. This aqueous dispersion is added to the oily phase containing resinoid and emulsifying ointment in hot condition while under stirring for 1hour at 10,000 rpm until a cream consistency is obtained.

### EXAMPLE - 5

| | |
|---|---|
| Tridax procumbens powder | 5% |
| Gum Olibanum resinoid (Ethyl alcohol extract) | 5% |
| Tulsi Oil (Ocimum sanctum) | 0.5% |
| Salicylic acid | 2% |
| Methyl paraben | 0.15% |
| Propyl paraben | 0.15% |
| Propylene glycol | 2% |
| Emulsifying ointment | 25% |
| Water | q.s. to make 100% |

The leaves of Tridax procumbens were shade dried for 48hours at room temperature. The crushed leaves were then soaked with water for 72hours at room temperature. At the end of this period water is decanted and then concentrated to 100ml by evaporating under vacuum. This concentrated solution is then lyophyllised to obtain a powder.

The leaves of Tulsi (Ocimum sanctum) were shade dried for 48hours and the dried leaves (1Kg) were steam distilled to get the essential oil (yield 0.3 to 0.4%).

Gum Olibanum lumps were powdered in 5Kg lots in an edge runner mill for 30 minutes. The powdered raw Gum Olibanum (250g) was then extracted at 30°C using ethyl alcohol (1.5litres) as solvent in a vertical churner for 8 hours. The solvent containing resinoid was then decanted and solvent was distilled off at atmospheric pressure. The resinoid (120g) obtained will have a density 0.940g/cm³ and surface tension (N/m) 0.300.

Weighed quantities of Gum Olibanum resinoid, Tulsi oil, propyl paraben and emulsifying ointment are taken in a container and the mixture is heated until all the ingredients melt. Tridax procumbens leaf extract powder, salicylic acid, methyl paraben and propylene glycol are taken along with water in a separate container and were homogenised until a homogenous dispersion is obtained. This aqueous dispersion is heated to the same temperature as that of the molten oily phase containing emulsifying ointment and other ingredients. The aqueous phase is added to the oily phase while in hot condition and dispersed using a homogeniser at 10,000 rpm for 2-3 hours, until a cream consistency is obtained.

### EXAMPLE - 6

| | |
|---|---|
| Diclofenac sodium | 2% |
| Gum Olibanum Resinoid (Methyl alcohol extract) | 4% |
| Emulsifying ointment | 29% |
| p-Chlorocresol | 0.15% |
| Water | q.s. to make 100% |

Gum olibanum lumps were powdered in 5kg lots in an edge runner for 30minutes. The powdered raw gum olibanum (250g) was extracted at 30°C using methyl alcohol (1.5 l.) as solvent in a vertical churner for 8 hours. The solvent containing resinoid was then decanted and distilled off at atmospheric pressure. The resinoid (120g) was obtained having density 0.940g/cm³ and surface tension (N/m) 0.300.

Weighed quantities of Gum Olibanum resinoid, emulsifying ointment and p-chlorocresol were melted until the mixture liquefies (oil phase). Diclofenac sodium was dissolved in water and heated to the same temperature as that of oil phase. The aqueous phase was then added to the oil. phase while stirring in a homogenizer at 10,000rpm, until the mass congeals and gives cream consistency.

### EXAMPLE - 7

| | |
|---|---|
| Piroxicam | 1% |
| Gum Olibanum resinoid (Methyl alcohol extract) | 7% |
| Aloe vera | 5% |
| Emulsifying ointment | 26% |
| Glycerine | 2% |
| Propyl Paraben | 0.15% |
| Methyl Paraben | 0.15% |
| Water | q.s. to make 100% |

Gum Olibanum lumps were powdered in 5 kg lots in an edge runner for 30 minutes. The powdered raw Gum Olibanum (250g) was extracted with ethyl alcohol (1.5l) at 30°C using a vertical churner for 8 hours. The solvent containing resinoid was then decanted and distilled off at atmospheric pressure to obtain pure resinoid.

Weighed quantities of Piroxicam, Gum Olibanum resinoid, dried juice of Aloe vera and emulsifying ointment are taken in a clean vessel along with propyl paraben and melted while stirring until a homogenous molten dispersion is obtained (oil phase).

Required quantities of water, methyl paraben and glycerine were taken in a separate vessel and the mixture is heated to the same temperature as that of oily phase. The aqueous phase was then added to oily phase in hot condition and mixed in a homogenizer for 1-3hours at 10,000 rpm, until the resultant mass congeals to cream consistency.

### EXAMPLE - 8

| | |
|---|---|
| Tridax Procumbens (Methyl alcohol extract) | 5% |
| Aloe Vera | 4% |
| Gum Olibanum Powder | 5% |
| Emulsifying ointment | 30% |
| p-Chlorocresol | 0.15% |
| Water | q.s. to make 100% |

The leaves of Tridax Procumbens were dried under shade for 72hours. The dried leaves were powdered, 300 g of powder was extracted with 11 of methanol in a soxhlet extractor for 6hours. The methanolic extract was filtered and evaporated to dryness using Rotavapor.

The naturally occurring Gum Olibanum exudate in dry state is taken as it is. The lumps (1kg) were powdered in an edge runner for 30minutes. The powdered raw gum was passed through 100 mesh screen. The methanolic extract of Tridax procumbens, the dried juice of aloe vera, emulsifying ointment and p-chlorocresol were taken in a vessel and heated while stirring until a molten homogenous dispersion is obtained (oily phase). Weighed quantity of Gum olibanum is dispersed in water and this dispersion is heated to the same temperature as that of oily phase. The aqueous phase is then added to the oily phase and stirred at 10,000rpm using a homogenizer, until the resultant mass congeals to cream consistency.

### EXAMPLE - 9

| | |
|---|---|
| Gum Olibanum powder | 5% |
| Aloe vera | 4% |
| Emulsifying ointment | 31% |
| Glycerine | 3% |
| p-Chlorocresol | 0.15% |
| Water | q.s. to make 100% |

The naturally occurring Gum Olibanum exudate in dry state is taken as it is. The lumps (1kg) were powdered in an edge runner for 30 minutes. The powdered raw gum olibanum was passed through 100 mesh sieve. The expressed juice of aloe vera is dried and dried juice is used in this formulation.

The dried juice of aloe vera, emulsifying ointment and p-chlorocresol were taken in a vessel and melted while stirring occasionally (oily phase) to give a homogenous dispersion. Gum Olibanum powder was dispersed in water along with glycerine. This dispersion was brought to the same temperature as that of oily phase. The aqueous dispersion was added to the oily phase while in hot condition and mixed thoroughly at 10,000rpm using homogenizer, until the mass congeals to cream consistency.

### EXAMPLE- 10

| | |
|---|---|
| Gum Olibanum resinoid (Methyl alcohol extract) | 5% |
| Aloe vera | 3% |
| Garlic (Allium sativum) Oil | 2% |
| Glycerine | 2% |
| p-Chlorocresol | 0.15% |
| Emulsifying ointment | 26% |
| Water | q.s. to make 100% |

Gum Olibanum lumps were powdered in 5 kg lots in an edge runner for 30minutes. The powdered raw Gum Olibanum (250g) was extracted at 30°C using methyl alcohol (1.5l). The solvent containing resinoid was decanted and distilled off at atmospheric pressure to obtain the resinoid.

Weighed quantities of, Gum Olibanum resinoid, dried juice of Aloe vera, garlic oil and emulsifying ointment were taken in a clean vessel along with p-chlorocresol. The ingredients were melted while mixing to get a homogeneous molten dispersion (oily phase). Specified quantity of glycerine was added to water and this aqueous phase was brought to the same temperature as that of oily phase. The aqueous phase was added to the oily phase in hot condition and mixed thoroughly at 10,000rpm using a homogeniser, until the resultant mass congeals and solidifies to cream consistency.

### EXAMPLE - 11

| | |
|---|---|
| Gum Olibanum powder | 5% |
| Salicylic Acid | 3% |
| Aloe Vera | 3% |
| Glycerine | 2% |
| Emulsifying ointment | 27% |
| p-Chlorocresol | 0.15% |
| Water | q.s. to make 100% |

The naturally occurring Gum Olibanum exudate is taken as it is. The lumps (1kg) were powdered in an edge runner for 30minutes. The powdered raw gum olibanum was passed through 100 mesh screen.

Weighed quantities of salicylic acid dried juice of aloe vera, emulsifying ointment and p-chlorocresol were taken in a clean vessel. The ingredients were melted while stirring . Gum Olibanum powder was dispersed in water along with glycerine. The temperature of this aqueous dispersion was brought to that of molten oily phase. The aqueous dispersion was added to the 'oily phase in hot condition and mixed thoroughly at 10,000rpm using a homogenizer until the mass congeals and solidifies to cream consistency.

### EXAMPLE - 12

| | |
|---|---|
| Tridax Procumbens (water extract) | 5% |
| Aloe vera | 3% |
| Garlic Oil (Allium sativum) | 2% |
| Emulsifying ointment | 26% |
| Glycerine | 2% |
| p-Chlorocresol | 0.15% |
| Water | q.s. to make 100% |

The leaves of Tridax procumbens were dried under shade for 72 hours. The leaves were crushed to powder (500g) and soaked with water for a week at room temperature. Water is decanted and concentrated to 100 ml by evaporation. This concentrated solution was then lyophilized to a powder.

Weighed quantities of emulsifying ointment, dried juice of aloe vera, Garlic oil and p-chlorocresol were taken in a clean vessel and melted while stirring until a homogenous molten mixture is obtained (oil phase). The freeze dried leaf extract of Tridax procumbens was dissolved in water along with glycerine. The temperature of this aqueous solution was brought to that of oily phase . The aqueous phase was then added to oily phase in hot condition and stirred at 10,000rpm using homogenizer, until the resultant mass congeals and solidifies to cream consistency.

### EXAMPLE - 13

| | |
|---|---|
| Gum Olibanum powder | 5% |
| Tridax procumbens (water extract) | 4% |
| Aloe vera | 5% |
| Turmeric powder | 0.5% |
| Glycerine | 3% |
| p-Chlorocresol | 0.15% |
| Emulsifying ointment | 25% |
| water | q.s. to make 100% |

The naturally occurring Gum Olibanum exudate in dry state is taken as it is. The lumps (1kg) were powdered in an edge runner for 30minutes. The powdered raw gum was passed through 100 mesh screen.

The leaves of Tridax procumben were dried under shade at room temperature for 72hours. The leaves were crushed to powder and the powder (500g) was soaked with water (11) for a week. Water is then decanted, filtered and concentrated to 100ml. The concentrated solution was then lyophilized to get powder.

Weighed quantities of emulsifying ointment, dried juice of aloe vera, and p-chlorocresol were taken in a clean vessel and the mixture was melted while stirring to get a molten homogenous mixture.

Gum Olibanum powder, dry powder of tridax procumbens were dispersed in water along with turmeric powder and glycerine. The temperature of this aqueous dispersion was brought to that of oily phase. The aqueous phase was then added to the oily phase in hot condition, while stirring at 10,000rpm using homogenizer, until the resultant mass congeals and solidifies to cream consistency.

### EXAMPLE - 14

| | |
|---|---|
| Tridax Procumbens (Water extract) | 5% |
| Aloe vera | 4% |
| Carbopol 934 | 3% |
| Methyl Paraben | 0.15% |
| Propyl Paraben | 0.15% |
| Manoethanolamine | q.s to bring pH to 6 |
| Propyleneglycol: water(50:50) | q.s to make 100% |

The leaves of Tridax procumbens were dried under shade for 72hours. The dried leaves were crushed to obtain powder. The crushed leaf powder(250g) was soaked in water (11) for a week.

Water is then decanted and concentrated to 100ml by evaporating under vacuum at room temperature. The concentrated solution is then lyophilized to obtain powder.

The dried leaf extract of Tridax Procumbens, the dried juice of aloe vera, propyl paraben were dissolved in pure propylene glycol by vigorous mixing using a stirrer. Carbopol 934 was dispersed separately in propylene glycol and water mixture (50:50) along with methyl paraben in another vessel. The mixture is stirred continuously for 1-3hours at 300rpm using mechanical stirrer or homogenizer. The pure propylene glycol solution containing tridax procumbens, aloe vera and propyl paraben was then added and stirring was continued for 1hour until a gel preparation was obtained. The pH of this gel is adjusted to 6.0 using monoethanol amine. The final gel preparation will be clear, transparent and non-sticky.

### EXAMPLE-15

| | |
|---|---|
| Gum Olibanum Meal (Resinoid free Gum Olibanum) | 9% |
| Aloe Vera | 5% |
| Salicylic Acid | 2% |
| Emulsifying ointment | 25% |
| Methyl paraben | 0.15% |
| Propyl paraben | 0.15% |
| Water | q.s. to make 100% |

Weighed quantities of Gum Olibanum Meal (resinoid free), Aloe Vera, Emulsifying ointment and Propyl paraben were taken in a tarred vessel and melted while stirring (Oily phase). Specified quantities of salicylic acid and methyl paraben were dispersed in water with the aid of heating until the temperature reaches to that of oily phase. This dispersion is added to molten oily phase while stirring at 10,000 rpm using homogenizer until the mass congeals and solidifies to cream consistency.

### EXAMPLE-16

| | |
|---|---|
| Gum Olibanum Meal (Resinoid free) | 9% |
| Tridax procumbens | 5% |
| Turmeric Powder | 0.5% |
| p-Chlorocresol | 0.15% |
| Propylene glycol | 2% |
| Emulsifying ointment | 27% |
| Water | q.s. to make 100% |

Weighed quantities of Gum Olibanum Meal (resinoid free), Emulsifying ointment and p-Chlorocresol were taken in a clean tarred vessel and melted (Oily phase).

The leaves of Tridax procumbens were shade dried for 48 hours, at room temperature. The crushed leaves (500g) were then soaked with water (1 liter) for 72hours at room temperature. At the end of this period, water is decanted and then concentrated to 100ml by evaporating under vacuum at room temperature. This concentrated solution is then lyophilised to obtain powder.

Tridax procumbens extract and Turmeric powder were dispersed in water along with Propylene glycol. The temperature of this aqueous dispersion was brought to that of oily phase. This aqueous dispersion was added to molten oily phase and stirred continuously at 10,000 rpm until the mass congeals and solidifies to cream consistency.

The formulations have a varied degree of therapeutic activity on cracked heels, dry skin disorders, skin allergies, depigmentation on the surface of skin and anti-fungal action.

The formulations of the present invention are not a mere admixture resulting in mere aggregation of the properties of individual ingredients but in a synergistic mixture resulting in enhanced curative properties.

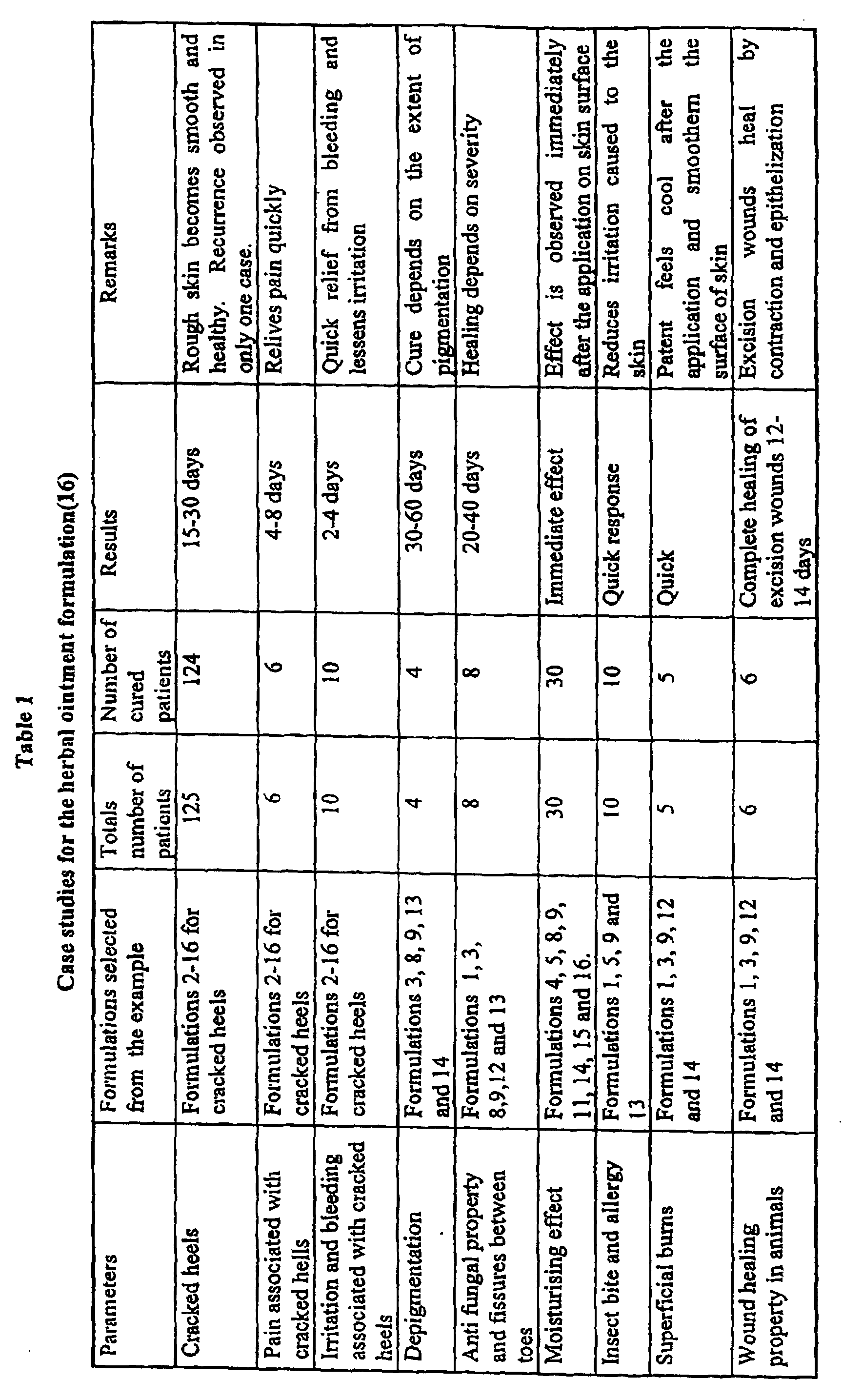

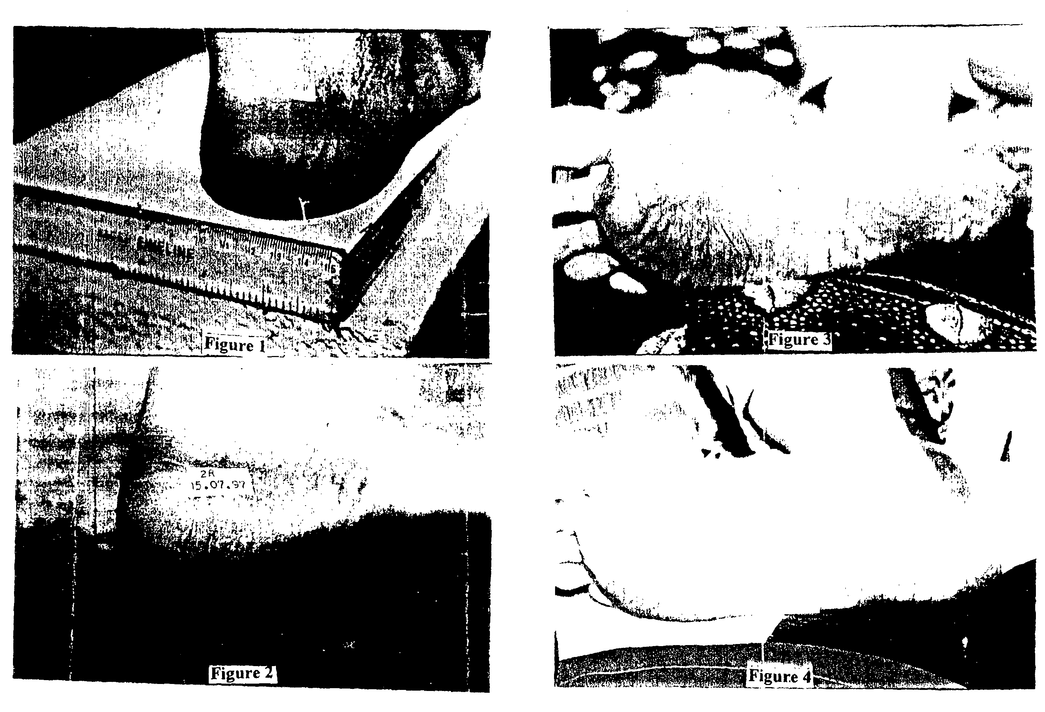

Figure 1 shows the treatment of a patient having cracked heel before the treatment. The herbal cream of the present patent application was applied twice daily (morning and evening) for about 17 days and it was observed that the cracked on the heel were cured as shown in figure 2 of the accompanying figures.

Likewise, another patient having badly cracked sole (Figure 3) was treated with the herbal composition of the present invention for about 21 days. The herbal cream was applied twice daily i.e. once in the morning and once in the evening after cleaning the sole with soap and water. The cracked sole was healed after the period as shown in figure 4 of the accompanying figures.

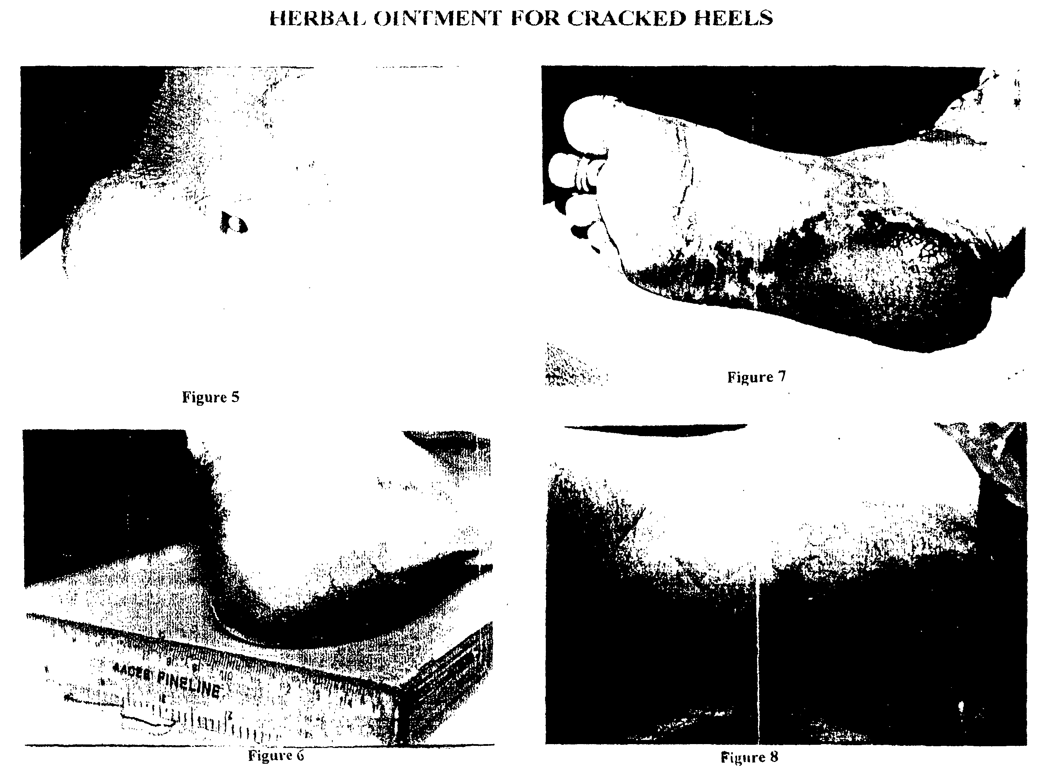

Another patient was treated for cracked heel (Figure 5) for 4 days with the herbal composition of the present invention. The patient's heel was cleaned with soap and water before applying the herbal cream twice daily. The cracks appeared on the sole were healed after the treatment as shown in figure 6 of the accompanying figures.

A female patient having badly cracked sole (Figure 7) was treated with the herbal composition of the present invention for 15 days. At the end of the treatment period, it was observed that cracks on the sole disappeared (Figure 8).

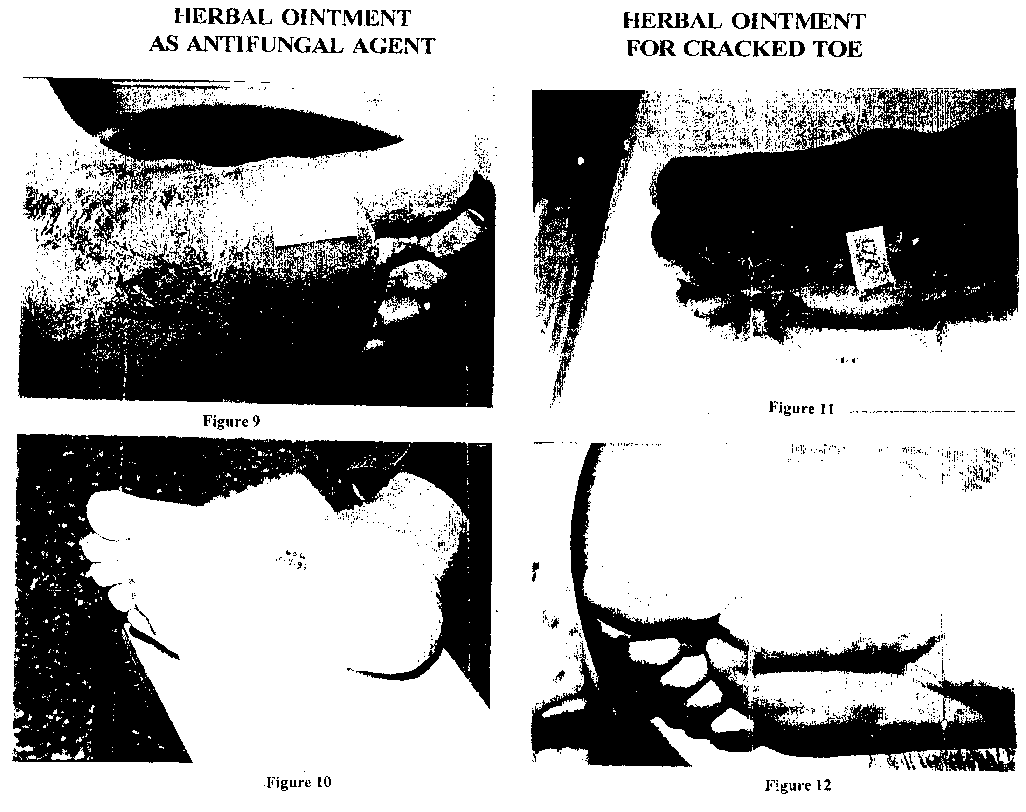

Yet another patient having a deep crack on a toe (Figure 9) was treated with the herbal composition for 17 days and at the end of the treatment, it was observed that the crack on a toe healed completed( Figure 10).

Another patient having fungal infection on sole (Figure 11) was treated with the herbal composition of the present invention for 50 days and during the course of the treatment, it was observed that the infection was gradually reduceing. At the end of the period of the treatment, the fungal infection on the sole has completely disappeared (Figure 12).

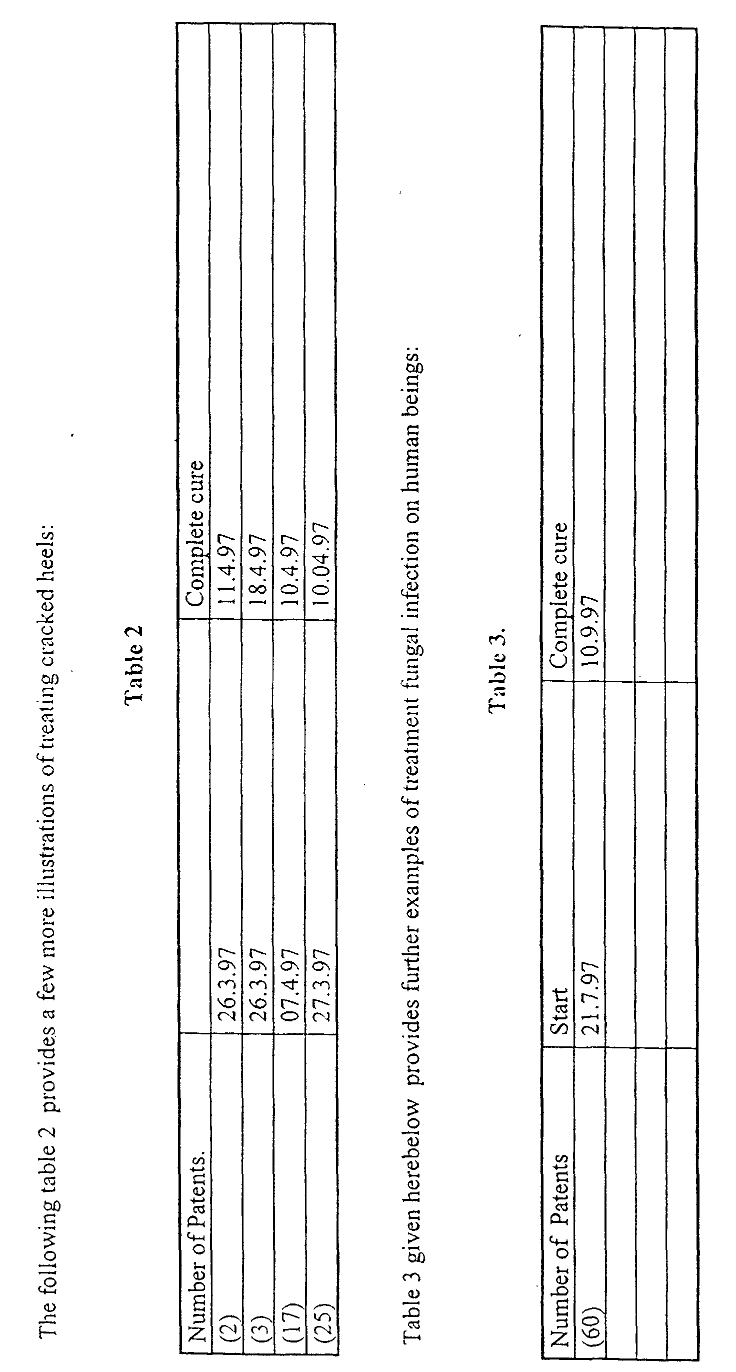

The following details provide data of clinical trials conducted in respect of the present invention. The herbal ointment of the present invention has been tried for healing of cracked heels, depigmentation of skin, skin rashes and fungal infections.

### Human studies:

### (1) Cracked heels:

The effect of the ointment is observed in terms closure of cracks relieving pain, bleeding in severe cracks and smoothening of hardened surface. The effect was observe within 2 days of application. The pain completely subsided within 3-4 days and surface becomes very smooth.

The reappearance of cracks was not observed after the discontinuation of medicine during the observation period of 12 months except in 3 cases our of 136 cases.

### (2) Depigmentation:

The black spot on the face, knee, forearm was completely cured in a period of 2 to 3 months in six individuals.

### (3) Skin rashes, minor cuts and bums:

The irritation caused by insect bite (Mosquito) was nullified by the ointment in couple of minutes in about 20 patients. It was also observed that the ointment is effective against minor cuts and bums.

### (4) Fungal infection:

In 6 cases the fungal infection involving irritation, painful open wounds on the foot and fissures in between fingers were subsided within 4 to 5 days and took about 2-3 months for the complete relief from open wounds and fissures.

### Animal studies:

The topical application on excision wounds on dorsal surface of wistar rats reduced the time taken for healing when compared to control wounds with respect to the parameters such as wound contraction, epithelisation and scar formation. The above study indicates that the ointment has pro healing property.

### ADVANGAGES

1. The present formulations, apart from healing the cracks on heels, are useful in arresting the bleeding due to cracks and reducing the pain.
2. Once the cracks on heels are cured, the recurrence of cracks is minimum, compared to commercially available products in the market.
3. The present formulations have moisturising effect on skin and hence can be used for dry skin disorders in cosmetic therapy.
4. The present formulations have antifungal activity and hence can be used for fungal infections on skin.
5. The present formulations provide excellent protection from darkening of skin due to minor cuts, bums, wounds and pimples.
6. The present formulations have good antiallergic activity in case of insect bites, rashes and reduce itching on the skin.
7. The present formulations can be used as base material in which ingredients have analgesic and antiinflammatory property can be incorporated for potentiation of their activities.

## Claims

1. A herbal formulation useful as a therapeutic and cosmetic applications for the treatment of general skin disorders comprising the following ingredients:
(a) at least **two** plant extracts in the form of oil or powder or mixtures thereof, the said plant extracts being selected from the group consisting of *Gymnena sylvestrae* water extract 3 to 6 wt%; *Tridax procumbens* water extract 3 to 6 wt%; its methanolic extract 4 to 6 wt%, *Allium sativum* oilhexane extract 1 to 3 wt %; dried juice of Aloe vera 2 to 6 wt %; Gum *Olibanum powder* in the natural form 4 to 7 wt%; *Gum Olibanum* resinoid or its organic extract 3 to 8 wt % and resinoid free *Gum Olibanum* meal 5 to 10 wt %;
(b) optionally, including any drug having anti-inflammatory and wound healing property or mixture thereof, the said drug being selected from the group consisting of Diclofenac sodium 1-3 wt.%, Salicyclic acid 1 to 4 wt%, Piroxicam 1 to 2 wt %, Turmeric powder 0.1 to 1 wt %;
(c) a gel base containing carbopol ranging between 1 to 4 wt % or an aqueous cream base containing emulsifying ointment ranging between 20 to 40 wt. %, preservatives ranging between 0.05-0.3% and a humectant ranging between 1-4 wt %; and
(d) remaining water to make 100 wt %.

2. A herbal formulation as claimed in claim 1 wherein preservatives used may be selected from the group consisting of methyl paraben, propyl paraben and p-chlorocresol.

3. A herbal formulation as claimed in claim 2 wherein methyl paraben and propyl paraben used as preservatives range between 0.1-0.3 wt%.

4. A herbal formulation as claimed in claim 2 wherein p-chlorocresol used as preservative ranges between 0.05-0.2%.

5. A herbal formulation as claimed in claim I wherein the gum olibanum resinoid hexane extract ranges between 4-6%.

6. A herbal formulation as claimed in claim 1 wherein gum olibanum resinoid methanol extract ranges between 3-8 wt%.

7. A herbal formulation as claimed in claim I wherein gum olibanum resinoid ethanol extract ranges between 4-6 wt%.

8. A herbal formulation as claimed in claim 1 wherein the general skin disorders are cracked heels, dicoloration, dry skin disorders, fungal infections and skin allergies.

9. A herbal formulation as claimed in claim 1 wherein glycerine is used as humectant and ranges between 1-4 wt%.

10. A herbal formulation as claimed in claim 1 wherein propylene glycol is used as humectant and ranges between 1-3 wt%.

11. A herbal formulation as claimed in claim 1 wherein the plant extracts used are obtained from plant material selected from *Gymnema sylvestrae* leaf (R.Br), *Gum olibanum, Tridax procumbens, Allium sativum, Aloe Vera.*

12. Use of herbal formulation as claimed in claim 1 for the manufacture of medicament for general skin disorders.

13. Use of herbal formulation as claimed in claim 1 for cosmetic applications.

14. A herbal formulation as claimed in claim 1 which is in the form of an emulsion of cream for application to the skin.

15. A herbal formulation as claimed in claim 1 useful in enhancing the healing of cracked heels with minimum recurrences.

16. A herbal formulation useful for enhancing healing of bums and wounds comprising the following ingredients.
(a) plant extracts in the form of powder or oil of medicinal plants, being *Tridax procumbens* water extract 3 to 6 wt. % and *gum olibanum* powder in the natural form 4-7 wt %.
(b) a gel base containing carbopol ranging between 1 to 4 wt % or an aqueous cream base containing emulsifying ointment ranging between 20 to 40 wt. % preservatives ranging between 0.05-0.3% and a humecant ranging between 1-4%;
(c) humectants selected from propylene glycol ranging between 1 to 3 wt % and glycerine ranging between 1 to 4 wt. %; and
(d) the balance being water to make 100 wt %.

17. An antifungal herbal formulation comprising the following ingredients:
(a) plant extracts in the form of powder or oil of medicinal plants, being *Tridax procumbens* water extract 3 to 6 wt % and *Gum Oilibanum* powder in the natural form 4 to 7 wt %;
(b) optionally any drug having anti-inflammatory and wound healing property or mixture thereof, the said drug being selected from drugs such as Diclofenac sodium 1-3 wt. %, Salicylic acid 1 to 4 wt %, Piroxicam 1 to 2 wt %, Turmeric powder 0.1 to 1 wt %;
(c) a gel base containing carbopol ranging between 1 to 4 wt % or an aqueous cream base containing emulsifying ointment ranging between 20 to 40 wt. %, preservatives ranging between 0.05-0.3% and humecant ranging between 1-4 wt. %.
(d) humectants selected from propylene glycol ranging between 1 to 3 wt. % and Glycerine ranging between 1 to 4 wt %; and
(e) the balance being water to make 100 wt %.

18. An anti allergic herbal formulation comprising the following ingredients:
a) plant extracts in the form of powder or oil of medicinal plants, being *Tridax procumbens* water extract 3 to 6 wt % and gum Olibanum powder in the natural form 4 to 7 %;
b) a gel base containing carbopol ranging between 1 to 4 wt % or an aqueous cream base containing emulsifying ointment ranging between 20 to 40 wt %, preservatives ranging between 0.05-0.3% and a humecant ranging between 1-4 wt %.
c) humectants selected from propylene glycol ranging between 1 to 3 wt % and Glycerine ranging between 1 to 4 wt % and
d) the balance being water to make 100 wt %.

19. A herbal formulation useful for cosmetic applications as moisturizer comprising the following ingredients:
a) at least two plant extracts in the form of powder or oil of medicinal plants, being selected from Gymnena sylvestrae water extract 3 to 6 wt %. *Aloe vera* (dried juice) 2 to 6 wt %, *Tridax procumbens* water extract 3 to 6 wt %, *Gum Oilbanum* meal and *Gum Olibanum* powder;
b) optionally any drug having anti-inflammatory and wound healing property or mixture thereof, the said drug being selected from group consisting of Diclofenac sodium 1-3 wt %, salicylic acid 1 to 4 wt %, Piroxicam 1 to 2 wt %, Turmeric powder 0.1 to 1 wt %.
c) a gel base containing carbopol ranging between 1 to 4 wt % or an aqueous cream base containing emulsifying ointment ranging between 20 to 40 wt %, preservatives ranging between 0.05-0.3 % and a humectant ranging between 1-4 wt%;
d) humectants selected from propylene glycol ranging between 1 to 3 wt % and Glycerine ranging between 1 to 4 wt %; and
e) the balance being water to make 100 wt %

20. A herbal formulation useful for therapeutic and cosmetic applications particularly discoloration comprising the following ingredients:
a) plant extracts in the form of powder or oil of medicinal plants, being *Gum Olibanum* resinoid, *Tridax procumbens* water extract 3 to 6 wt %, *Gum Olibanum* powder;
b) a gel base containing carbopol ranging between 1 to 4 wt % or an aqueous cream base containing emulsifying ointment ranging between 20 to 40 wt % preservatives ranging between 0.05-0.3 % and ahumectant ranging between 1-4 %.
c) humectants selected from propylene glycol ranging between I to 3 wt %, and Glycerine ranging between 1 to 4 wt %; and
d) the balance being water to make 100 wt %.

## Patentansprüche

1. Kräuterformulierung, welche als therapeutische und kosmetische Anwendungen zur Behandlung allgemeiner Hautleiden nützlich ist, umfassend die folgenden Bestandteile:
(a) zumindest **zwei** Pflanzenextrakte in Form eines Öls oder Pulvers oder Mischungen davon, wobei die Pflanzenextrakte ausgewählt sind aus der Gruppe, bestehend aus 3 bis 6 Gew.% wässrigem *Gymnena sylvestrae*-Extrakt, 3 bis 6 Gew.% wässrigem *Tridax procumbens-Extrakt,* 4 bis 6 Gew.% von dessen methanolischem Extrakt, 1 bis 3 Gew.% *Allium sativum*-Ölhexanextrakt; 2 bis 6 Gew.% Aloe-Vera-Trockensaft; 4 bis 7 Gew.% *Gum olibanum*-Pulver in der natürlichen Form; 3 bis 8 Gew.% *Gum olibanum*-Resinoid oder dessen organischem Extrakt und 5 bis 10 Gew.% Resinoidfreiem *Gum olibanum*-Mehl;
(b) gegebenenfalls einschließend ein Arzneimittel mit entzündungshemmender und wundheilender Eigenschaft oder eine Mischung davon, wobei das Arzneimittel ausgewählt ist aus der Gruppe, bestehend aus 1 bis 3 Gew.% Diclofenac-Natrium, 1 bis 4 Gew.% Salicylsäure, 1 bis 2 Gew.% Piroxicam, 0,1 bis 1 Gew.% Curcuma-Pulver;
(c) eine Gelbasis, enthaltend Carbopol in einer Menge zwischen 1 und 4 Gew.%, oder eine wässrige Cremebasis, enthaltend eine emulgierende Salbe in einer Menge zwischen 20 und 40 Gew.%, Konservierungsmittel in einer Menge zwischen 0,05 und 0,3% und ein Benetzungsmittel in einer Menge zwischen 1 und 4 Gew.%; und
(d) übriges Wasser, um 100 Gew.% zu ergeben.

2. Kräuterformulierung, wie in Anspruch 1 beansprucht, wobei die verwendeten Konservierungsmittel aus der Gruppe, bestehend aus Methylparaben, Propylparaben und p-Chlorkresol, ausgewählt sein können.

3. Kräuterformulierung, wie in Anspruch 2 beansprucht, wobei die Menge an Methylparaben und Propylparaben, die als Konservierungsmittel verwendet werden, zwischen 0,1 und 0,3 Gew.% liegt.

4. Kräuterformulierung, wie in Anspruch 2 beansprucht, wobei die Menge des als Konservierungsmittel verwendeten p-Chlorkresols zwischen 0,05 und 0,2% liegt.

5. Kräuterformulierung, wie in Anspruch 1 beansprucht, wobei die Menge an Gum olibanum-Resinoidhexanextrakt zwischen 4 und 6% liegt.

6. Kräuterformulierung, wie in Anspruch 1 beansprucht, wobei die Menge an Gum olibanum-Resinoidmethanolextrakt zwischen 3 und 8 Gew.% liegt.

7. Kräuterformulierung, wie in Anspruch 1 beansprucht, wobei die Menge an Gum olibanum-Resinoidethanolextrakt zwischen 4 und 6 Gew.% liegt.

8. Kräuterformulierung, wie in Anspruch 1 beansprucht, wobei die allgemeinen Hautleiden rissige Fersen, Verfärbungen, Störungen aufgrund trockener Haut, Pilzinfektionen und Hautallergien sind.

9. Kräuterformulierung, wie in Anspruch 1 beansprucht, wobei Glycerin als Benetzungsmittel verwendet wird und in einer Menge zwischen 1 und 4 Gew.% vorkommt.

10. Kräuterformulierung, wie in Anspruch 1 beansprucht, wobei Propylenglycol als Benetzungsmittel verwendet wird und in einer Menge zwischen 1 und 3 Gew.% vorkommt.

11. Kräuterformulierung, wie in Anspruch 1 beansprucht, wobei die verwendeten Pflanzenextrakte aus Pflanzenmaterial erhalten werden, das ausgewählt ist aus *Gymnema sylvestrae*-Blättern (R.Br.), *Gum olibanum, Tridax Procumbens, Allium sativum, Aloe vera*.

12. Verwendung einer Kräuterformulierung, wie in Anspruch 1 beansprucht, zur Herstellung eines Medikaments für allgemeine Hautleiden.

13. Verwendung einer Kräuterformulierung, wie in Anspruch 1 beansprucht, für kosmetische Anwendungen.

14. Kräuterformulierung, wie in Anspruch 1 beansprucht, welche in Form einer Creme-Emulsion zum Auftragen auf die Haut vorliegt.

15. Kräuterformulierung, wie in Anspruch 1 beansprucht, welche zur Verbesserung der Heilung rissiger Fersen mit einem Mindestmaß an Rückfällen nützlich ist.

16. Kräuterformulierung, welche zur Verbesserung der Heilung von Verbrennungen und Wunden nützlich ist, umfassend die folgenden Bestandteile:
(a) Pflanzenextrakte in Form eines Pulvers oder Öls von Heilpflanzen, wobei es sich um 3 bis 6 Gew.% wässrigen *Tridax procumbens*-Extrakt und 4 bis 7 Gew.% *Gum olibanum*-Pulver in der natürlichen Form handelt;
(b) eine Gelbasis, enthaltend Carbopol in einer Menge zwischen 1 und 4 Gew.%, oder eine wässrige Cremebasis, enthaltend eine emulgierende Salbe in einer Menge zwischen 20 und 40 Gew.%, Konservierungsmittel in einer Menge zwischen 0,05 und 0,3% und ein Benetzungsmittel in einer Menge zwischen 1 und 4%;
(c) Benetzungsmittel, die ausgewählt sind aus Propylenglycol in einer Menge zwischen 1 und 3 Gew.% und Glycerin in einer Menge zwischen 1 und 4 Gew.%; und
(d) wobei der Rest Wasser ist, um 100 Gew.% zu ergeben.

17. Pilztötende Kräuterformulierung, umfassend die folgenden Bestandteile:
(a) Pflanzenextrakte in Form eines Pulvers oder Öls von Heilpflanzen, wobei es sich um 3 bis 6 Gew.% wässrigen *Tridax procumbens-Extrakt* und 4 bis 7 Gew.% *Gum olibanum*-Pulver in der natürlichen Form handelt;
(b) gegebenenfalls jedes Arzneimittel mit entzündungshemmender und wundheilender Eigenschaft oder eine Mischung davon, wobei das Arzneimittel ausgewählt ist aus Arzneimitteln wie z.B. 1 bis 3 Gew.% Diclofenac-Natrium, 1 bis 4 Gew.% Salicylsäure, 1 bis 2 Gew.% Piroxicam, 0,1 bis 1 Gew.% Curcuma-Pulver;
(c) eine Gelbasis, enthaltend Carbopol in einer Menge zwischen 1 und 4 Gew.%, oder eine wässrige Cremebasis, enthaltend eine emulgierende Salbe in einer Menge zwischen 20 und 40 Gew.%, Konservierungsmittel in einer Menge zwischen 0,05 und 0,3% und ein Benetzungsmittel in einer Menge zwischen 1 und 4 Gew.%;
(d) Benetzungsmittel, die ausgewählt sind aus Propylenglycol in einer Menge zwischen 1 und 3 Gew.% und Glycerin in einer Menge zwischen 1 und 4 Gew.%; und
(e) wobei der Rest Wasser ist, um 100 Gew.% zu ergeben.

18. Antiallergische Kräuterformulierung, umfassend die folgenden Bestandteile:
(a) Pflanzenextrakte in Form eines Pulvers oder Öls von Heilpflanzen, wobei es sich um 3 bis 6 Gew.% wässrigen *Tridax procumbens-Extrakt* und 4 bis 7% *Gum olibanum*-Pulver in der natürlichen Form handelt;
(b) eine Gelbasis, enthaltend Carbopol in einer Menge zwischen 1 und 4 Gew.%, oder eine wässrige Cremebasis, enthaltend eine emulgierende Salbe in einer Menge zwischen 20 und 40 Gew.%, Konservierungsmittel in einer Menge zwischen 0,05 und 0,3% und ein Benetzungsmittel in einer Menge zwischen 1 und 4 Gew.%;
(c) Benetzungsmittel, die ausgewählt sind aus Propylenglycol in einer Menge zwischen 1 und 3 Gew.% und Glycerin in einer Menge zwischen 1 und 4 Gew.%; und
(d) wobei der Rest Wasser ist, um 100 Gew.% zu ergeben.

19. Kräuterformulierung, welche für kosmetische Anwendungen als Feuchtigkeitscreme nützlich ist, umfassend die folgenden Bestandteile:
(a) zumindest zwei Pflanzenextrakte in Form eines Pulvers oder Öls von Heilpflanzen, die ausgewählt sind aus 3 bis 6 Gew.% wässrigem *Gymnena sylvestrae*-Extrakt, 2 bis 6 Gew.% Aloe-Vera-(Trockensaft), 3 bis 6 Gew.% wässrigem *Tridax procumbens-Extrakt, Gum olibanum*-Mehl und *Gum olibanum*-Pulver;
(b) gegebenenfalls jedes Arzneimittel mit entzündungshemmender und wundheilender Eigenschaft oder eine Mischung davon, wobei das Arzneimittel ausgewählt ist aus der Gruppe, bestehend aus 1 bis 3 Gew.% Diclofenac-Natrium, 1 bis 4 Gew.% Salicylsäure, 1 bis 2 Gew.% Piroxicam, 0,1 bis 1 Gew.% Curcuma-Pulver;
(c) eine Gelbasis, enthaltend Carbopol in einer Menge zwischen 1 und 4 Gew.%, oder eine wässrige Cremebasis, enthaltend eine emulgierende Salbe in einer Menge zwischen 20 und 40 Gew.%, Konservierungsmittel in einer Menge zwischen 0,05 und 0,3% und ein Benetzungsmittel in einer Menge zwischen 1 und 4 Gew.%;
(d) Benetzungsmittel, die ausgewählt sind aus Propylenglycol in einer Menge zwischen 1 und 3 Gew.% und Glycerin in einer Menge zwischen 1 und 4 Gew.%; und
(e) wobei der Rest Wasser ist, um 100 Gew.% zu ergeben.

20. Kräuterformulierung, welche für therapeutische und kosmetische Anwendungen, insbesondere bei Verfärbungen, nützlich ist, umfassend die folgenden Bestandteile:
(a) Pflanzenextrakte in Form eines Pulvers oder Öls von Heilpflanzen, wobei es sich um *Gum olibanum*-Resinoid, 3 bis 6 Gew.% wässrigen *Tridax procumbens*-Extrakt, *Gum olibanum*-Pulver handelt;
(b) eine Gelbasis, enthaltend Carbopol in einer Menge zwischen 1 und 4 Gew.%, oder eine wässrige Cremebasis, enthaltend eine emulgierende Salbe in einer Menge zwischen 20 und 40 Gew.%, Konservierungsmittel in einer Menge zwischen 0,05 und 0,3% und ein Benetzungsmittel in einer Menge zwischen 1 und 4%;
(c) Benetzungsmittel, die ausgewählt sind aus Propylenglycol in einer Menge zwischen 1 und 3 Gew.% und Glycerin in einer Menge zwischen 1 und 4 Gew.%; und
(d) wobei der Rest Wasser ist, um 100 Gew.% zu ergeben.

## Revendications

1. Formulation à base de plantes utilisable dans des applications thérapeutiques et cosmétiques pour le traitement d'affections dermatologiques en général, comprenant les ingrédients suivants :
(a) au moins **deux** extraits végétaux sous forme d'huile ou de poudre ou leurs mélanges, lesdits extraits végétaux étant choisis dans le groupe constitué d'extrait aqueux de *Gymnena sylvestrae* à 3 à 6 % en poids ; d'extrait aqueux de *Tridax procumbens* à 3 à 6 % en poids ; de son extrait méthanolique à 4 à 6 % en poids ; d'extrait d'hexane huileux de *Allium sativum* à 1 à 3 % en poids; de suc déshydraté d'Aloe vera à 2 à 6 % en poids; de poudre de *Gum Olibanum* sous sa forme naturelle à 4 à 7 % en poids; de résinoïde de *Gum Olibanum* ou son extrait organique à 3 à 8 % en poids et de farine de *Gum Olibanum* exempte de résinoïde à 5 à 10 % en poids;
(b) comprenant facultativement tout agent thérapeutique possédant une propriété anti-inflammatoire et cicatrisante ou leur mélange, ledit agent thérapeutique étant choisi dans le groupe constitué de sodium de diclofenac à 1 à 3 % en poids, d'acide salicylique à 1 à 4 % en poids, de piroxicam à 1 à 2 % en poids et de poudre de turmeric à 0,1 à 1 % en poids ;
(c) une base en gel contenant du carbopol à une teneur comprise entre 1 et 4 % en poids ou une base en crème aqueuse contenant un onguent émulsifiant à une teneur comprise entre 20 et 40 % en poids, des conservateurs à une teneur comprise entre 0,05 et 0,3 % et un agent humectant à une teneur comprise entre 1 et 4 % en poids ; et
(d) le reste étant constitué d'eau pour atteindre 100 % en poids.

2. Formulation à base de plantes selon la revendication 1, dans laquelle les conservateurs utilisés peuvent être choisis dans le groupe constitué de méthyl parabène, de propyl parabène et de p-chlorocrésol.

3. Formulation à base de plantes selon la revendication 2, dans laquelle le méthyl parabène et le propyl parabène, utilisés comme conservateurs, sont compris à une teneur de 0,1 à 0,3 % en poids.

4. Formulation à base de plantes selon la revendication 2, dans laquelle le p-chlorocrésol, utilisé comme conservateur, est compris à une teneur de 0,05 à 0,2 % en poids.

5. Formulation à base de plantes selon la revendication 1, dans laquelle l'extrait d'hexane résinoïde de *Gum Olibanum* est compris à une teneur de 4 à 6 % en poids.

6. Formulation à base de plantes selon la revendication 1, dans laquelle l'extrait de méthanol résinoïde de Gum *Oliba*num est compris à une teneur de 3 à 8 % en poids.

7. Formulation à base de plantes selon la revendication 1, dans laquelle l'extrait d'éthanol résinoïde de *Gum Olibanum* est compris à une teneur de 4 à 6 % en poids.

8. Formulation à base de plantes selon la revendication 1, dans laquelle les affections dermatologiques en général sont les talons crevassés, la décoloration, les affections liées aux peaux desséchées, les infections fongiques et les allergies dermatologiques.

9. Formulation à base de plantes selon la revendication 1, dans laquelle la glycérine est utilisée comme agent humectant et est comprise à une teneur de 1 à 4 % en poids.

10. Formulation à base de plantes selon la revendication 1, dans laquelle le propylène glycol est utilisé comme agent humectant et est compris à une teneur de 1 à 3 % en poids.

11. Formulation à base de plantes selon la revendication 1, dans laquelle les extraits végétaux utilisés sont obtenus à partir de matériaux végétaux choisis parmi les feuille de *Gymnema sylvestrae* (R.Br), *Gum Olibanum, Tridax procumbens, Allium sativum* et *Aloe vera*.

12. Utilisation de la formulation à base de plantes selon la revendication 1 pour la fabrication d'un médicament pour les affections dermatologiques en général.

13. Utilisation de la formulation à base de plantes selon la revendication 1 pour des applications cosmétiques.

14. Formulation à base de plantes selon la revendication 1, qui se présente sous la forme d'une émulsion de crème pour application sur la peau.

15. Formulation à base de plantes selon la revendication 1, utile pour renforcer la cicatrisation des talons crevassés avec des récurrences minimales.

16. Formulation à base de plantes, utile pour renforcer la cicatrisation des brûlures et des plaies, comprenant les ingrédients suivants :
(a) des extraits végétaux sous forme de poudre ou d'huile de plantes médicinales qui sont constitués d'extrait aqueux de *Tridax procumbens* à 3 à 6 % en poids et de poudre de *Gum ilobanum* sous sa forme naturelle à 4 à 7 % en poids;
(b) une base en gel contenant du carbopol à une teneur comprise entre 1 et 4 % en poids ou une base en crème aqueuse contenant un onguent émulsifiant à une teneur comprise entre 20 et 40 % en poids, des conservateurs à une teneur comprise entre 0,05 et 0,3 % et un agent humectant à une teneur comprise entre 1 et 4 % en poids;
(c) des agents humectants choisis entre du propylène glycol à une teneur comprise entre 1 et 3 % en poids et de la glycérine à une teneur comprise entre 1 et 4 % en poids ; et
(d) l'équilibre étant constitué d'eau pour atteindre 100 % en poids.

17. Formulation antifongique à base de plantes comprenant les ingrédients suivants :
(a) des extraits végétaux sous forme de poudre ou d'huile de plantes médicinales, qui sont constitués d'extrait aqueux de *Tridax* procumbens à 3 à 6 % en poids et de poudre de *Gum Olibanum* sous sa forme naturelle à 4 à 7 % en poids,;
(b) comprenant facultativement tout agent thérapeutique possédant une propriété anti-inflammatoire et cicatrisante ou leur mélange, ledit agent thérapeutique étant choisi parmi des agents thérapeutiques tels que le sodium de diclofenac à 1 à 3 % en poids, l'acide salicylique à 1 à 4 % en poids, le piroxicam à 1 à 2 % en poids, la poudre de turmeric à 0,1 à 1 % en poids ;
(c) une base en gel contenant du carbopol à une teneur comprise entre 1 et 4 % en poids ou une base en crème aqueuse à une teneur comprise entre 20 et 40 % en poids d'un onguent émulsifiant, des conservateurs à une teneur comprise entre 0,05 et 0,3 %, et un agent humectant à une teneur comprise entre 1 et 4 % en poids ;
(d) des agents humectants choisis entre le propylène glycol à une teneur comprise entre 1 et 3 % en poids, la glycérine à une teneur comprise entre 1 et 4 % en poids ; et
(e) l'équilibre étant constitué d'eau afin d'atteindre 100 % en poids.

18. Formulation anti-allergique à base de plantes comprenant les ingrédients suivants :
(a) des extraits végétaux sous forme de poudre ou d'huile de plantes médicinales, qui sont l'extrait aqueux de *Tridax procumbens* à 3 à 6 % en poids et la poudre de *Gum Olibanum* sous forme naturelle à 4 à 7 % en poids ;
(b) une base en gel contenant du carbopol à une teneur comprise entre 1 et 4 % en poids ou une base en crème aqueuse contenant un onguent émulsifiant à une teneur comprise entre 20 et 40 % en poids, des conservateurs à une teneur comprise entre 0,05 et 0,3 % en poids et un agent humectant à une teneur comprise entre 1 et 4 % en poids ;
(c) des agents humectants choisis entre le propylène glycol à une teneur comprise entre 1 et 3 % et de la glycérine à une teneur comprise entre 1 et 4 % en poids ; et
(d) l'équilibre étant constitué d'eau afin d'atteindre 100 % en poids.

19. Formulation à base de plantes utile pour des applications cosmétiques telles que les crèmes hydratantes, comprenant les ingrédients suivants :
(a) au moins deux extraits végétaux sous forme de poudre ou d'huile de plantes médicinales, qui sont choisis parmi l'extrait aqueux de *Gymnena sylvestrae* à 3 à 6 % en poids, l'Aloe *vera* (suc déshydraté) à 2 à 6 % en poids ; l'extrait aqueux de *Tridax procumbens* à 3 à 6 % en poids, la farine de *Gum Olibanum* et la poudre de Gum *Olibanum* ;
(b) comprenant facultativement tout agent thérapeutique possédant une propriété anti-inflammatoire et cicatrisante ou leur mélange, ledit agent thérapeutique étant choisi dans le groupe constitué de sodium de diclofenac à 1 à 3 % en poids, d'acide salicylique à 1 à 4 % en poids, de piroxicam à 1 à 2 % en poids et de poudre de turmeric à 0,1 à 1 % en poids ;
(c) une base en gel contenant du carbopol à une teneur comprise entre 1 et 4 % en poids ou une base en crème aqueuse contenant un onguent émulsifiant à une teneur comprise entre 20 et 40 % en poids, des conservateurs à une teneur comprise entre 0,05 et 0,3 % en poids et un agent humectant à une teneur comprise entre 1 et 4 % en poids ;
(d) des agents humectants choisis parmi le propylène glycol à une teneur comprise entre 1 et 3 % en poids, et de la glycérine à une teneur comprise entre 1 et 4 % en poids ; et
(e) l'équilibre étant constitué d'eau afin d'atteindre 100 % en poids.

20. Formulation à base de plantes utilisable pour des applications thérapeutiques et cosmétiques, en particulier pour la décoloration, comprenant les ingrédients suivants :
(a) des extraits végétaux sous forme de poudre ou d'huile de plantes médicinales, qui sont le résinoïde de Gum *Olibanum*, l'extrait aqueux de *Tridax procumbens* à 3 à 6 % en poids et la poudre de *Gum Olibanum* ;
(b) une base en gel contenant du carbopol à une teneur comprise entre 1 et 4 % en poids ou une base en crème aqueuse contenant un onguent émulsifiant à une teneur comprise entre 20 et 40 % en poids, des conservateurs à une teneur comprise entre 0,05 et 0,3 % en poids et un agent humectant à une teneur comprise entre 1 et 4 % en poids ;
(c) des agents humectants choisis entre le propylène glycol à une teneur comprise entre 1 et 3 % en poids et la glycérine à une teneur comprise entre 1 et 4 % en poids ; et
(d) l'équilibre étant constitué d'eau afin d'atteindre 100 % en poids.
